# EUROPEAN PATENT SPECIFICATION

(11) **EP 3 632 497 B1**
(45) Date of publication and mention of the grant of the patent: **10.07.2024**
(21) Application number: 18198450.1
(22) Date of filing: 03.10.2018
(51) Int. Cl.: A61M 27/00, A61M 1/00, F16K 31/22, F16K 7/08, F16K 31/163, F16K 31/26

(54) **BODILY FLUID DRAINAGE SYSTEM WITH VOLUME LIMITING AND ADJUSTABLE VOLUME CAPACITY FUNCTIONALITY**
KÖRPERFLÜSSIGKEITENTWÄSSERUNGSSYSTEM MIT VOLUMENBEGRENZUNG UND EINSTELLBARER VOLUMENKAPAZITÄTSFUNKTIONALITÄT
SYSTÈME DE DRAINAGE DE FLUIDES CORPORELS À LIMITATION DE VOLUME ET À CAPACITÉ DE VOLUME RÉGLABLE

(43) Date of publication of application: 08.04.2020
(73) Proprietor: Integra LifeSciences Switzerland Sàrl, 2400 Le Locle (CH)
(72) Inventor: DEXTRADEUR, Alan, J., Plainsboro, NJ 08536 (US); DEFUSCO, Michael, A., Plainsboro, NJ 08536 (US); TRIGGER, Alyssa, R., Plainsboro, NJ 08536 (US)
(74) Representative: Carpmaels & Ransford LLP

(56) References cited:
- EP-A1- 2 926 858
- DE-U1- 9 409 627
- US-A- 3 965 903
- US-A- 5 090 443
- US-A- 5 669 892
- US-A1- 2009 088 710

## Description

### BACKGROUND OF THE INVENTION

### Field of the Invention

The present invention relates to a drainage system, in particular, an implantable system for the drainage of a bodily fluid such as cerebrospinal fluid (CSF). More particularly, the invention relates to an improved bodily fluid drainage system with volume limiting and adjustable volume capacity functionality.

### Description of Related Art

During treatment for medical disorders it is not uncommon to require the removal of excess fluid from the body. One common use of such bodily drainage systems is in the treatment of hydrocephalus, namely the build-up of CSF that surrounds the brain. Under normal conditions, the rate at which the CSF is produced is substantially equal to the rate at which it is absorbed by the body. Any excess build-up of CSF when this rate is no longer equal results in undesirable increased intracranial pressure (ICP) beyond normal physiological maximum pressure limits. During the treatment of hydrocephalus, a shunt is typically implanted in fluid communication with a ventricle within the brain to allow for drainage of the CSF at a desired rate. Maintaining the proper drainage flow rate is important. On the one hand, too fast a flow rate ("overdrainage") may result in severe headaches or collapse of one or more ventricular cavities within the brain. Whereas, on the other hand, too slow a flow rate ("underdrainage") may result in undesired intracranial pressure resulting in possible brain damage.

The rate of flow or drainage of the CSF is adjustable via a programmable valve having a plurality of different pressure settings. Desirably, such programmable pressure settings are adjustable non-invasively following implantation in the body. This way the pressure setting may be controlled over the duration that the drainage system is implanted in the body should conditions warrant adjustment. For example, commercially available implantable valves sold by Codman & Shurtleff, Inc. including the CODMAN^{®} HAKIM^{®} programmable valve (US Patent No. 4,595,390) or the CERTAS^{®} programmable valve (US Patent No. 8,322,365). In these external drainage systems, CSF is drained from the body into an external collection chamber. External drainage systems drain bodily fluid from the patient through a drainage catheter into an external sterile collection container (e.g., a bag) in which the fluid is collected. Hence, such external drainage system is a "closed" collection system (i.e., wherein the bodily fluid is not exposed to microbes in room air) to prevent infection. Overfilling of the collection container may undesirably leak fluid from the closed collection system resulting in faulty operation of the system upon becoming wet (e.g., if the filter becomes wet from the bodily fluid) and/or possible infection within the drainage system if the fluid becomes exposed to microbes in the room air.

Of course, visual monitoring of the level of bodily fluid collected is one way to prevent such overfilling of the collection container, however, this requires one-on-one monitoring by a medical professional who typically cannot devote such time to only one patient given the number of patients under their care to at any given time. It is therefore desirable to develop an overfill prevention system for a bodily fluid drainage system that does not require continuous visual monitoring by medical personnel.

US Patent No. 8,221,366 is directed to a volume limiting bodily fluid drainage system comprising a buoyant float hingedly connected with the top of the collection chamber. The buoyant float member has a lumen that is filed with air, gas or other buoyant material. This float member occupies a volume of about 10 ml to about 15 ml within the collection chamber. The collection chamber lumen is configured with a volume about equal to, or slightly larger than the float member volume plus the prescribed volume. The volume of the collection chamber exceeds the combined volumes of the float member and the prescribed volume by about 2 ml to about 10 ml. As a result, this patented float member undesirably takes up a significant volume within the collection chamber leaving only a small proportion of the volume of the collection chamber for fluid to be collected or otherwise requires a very large collection chamber sufficient to accommodate the combined volume required both for the float member and the capacity of fluid to be drained from the patient.

The configuration in US Patent No. 8,221,366 also disadvantageously requires that the float member be designed at its top portion to include a hollow chimney extension with a pressure equalization hole at the top. Such pressure equalization hole communicates with air inside the float member lumen permitting gases under pressure to flow into or out of the float member lumen through the pressure equalization hole. The pressure equalization hole equalizes pressure differences between the inside and outside of the float member during sterilization under pressure, thereby preventing damage to the float member during sterilization processing. The hollow chimney extension is expressly intended to position the equalization hole sufficiently high up in the collection chamber well above the collected fluid level, thereby preventing the collected fluid (e.g., CSF) from entering the float member lumen under normal operating conditions. Due to the fact that the buoyant float member is filled with air, gas or other buoyant material, it is important to avoid fluid from entering the float chamber lumen which could affect buoyancy and hence proper overfill prevention functionality. Thus, the volume of fluid collectable in the US Patent No. 8,221,366 apparatus is disadvantageously limited not only by the overall volume of the float member, as discussed in the preceding paragraph, but also by the required hollow chimney design with the hole at the top in which fluid must be prevented from entering. This patented assembly is also limited only to overfill prevention without permitting adjustment of the volume capacity of the collection chamber itself.

US5669892A relates to an overfill protection apparatus associated with a shutoff valve for a suction drainage system that prevents the overfilling of the waste fluid container. EP2926858A1 relates to tools to control drainage of bodily fluid from a patient and more particularly to external volume-limiting devices for withdrawing cerebrospinal fluid. US3965903A relates to medical suction bottle assemblies for effecting drainage, where the assembly has a float responsive to the level of drainage in the bottle for preventing overflow.

It is therefore desirable to develop an improved volume limiting bodily fluid drainage system in which the proportion of volume within the collection chamber taken up by the volume limiting mechanism is minimized and not susceptible to malfunction in the event of exposure to the bodily fluid. Moreover, it is desirable to develop an improved bodily fluid drainage system in which the volume capacity of the fluid collection chamber is adjustable in addition to the overfill prevention functionality.

### Summary of the Invention

The present inventive improved CSF drainage system includes an overfill prevention mechanism that automatically activates when a preset amount of fluid enters the collection chamber, increasing the safety of the patient as well as reducing the workload on hospital staff that otherwise would have to manually and frequently monitor the level.

The present invention, as claimed in the appended claim 1, provides an implantable bodily fluid drainage system including a collection vessel and a mechanical volume limiting mechanism for preventing overfill of the collection chamber. In contrast to convention mechanisms, the present inventive mechanical volume limiting mechanism is not susceptible to malfunction if immersed in the bodily fluid.

According to the invention, the drainage system relates to the collection vessel having an outer collection chamber and an inner collection chamber, wherein the inner collection chamber is axially displaceable within the outer collection chamber. The mechanical volume limiting mechanism includes a bent moveable arm rotatably disposed in the inner collection chamber and supported substantially at its center by a hinge to the fixed supporting arm. A stopper head is disposed at one terminating end of the bent moveable arm and a pinion gear disposed at an opposite terminating end of the bent moveable arm. A rack gear mounted to the inner surface of the inner collection chamber engages the pinion gear rotating the bent moveable arm about the hinge. As the bodily fluid accumulates in the inner collection chamber, the inner collection chamber travels axially downward relative to the outer collection chamber while the pinion gear on the bent moveable arm engages with the rack gear thereby raising the stopper head upwards, eventually closing off the inlet port of the outer collection chamber.

Still another aspect of the present drainage system relates to an implantable bodily fluid drainage system with an adjustable volume capacity mechanism for varying a maximum volume capacity of the inner collection chamber. The adjustable volume capacity mechanism includes a coil spring disposed in the outer collection chamber, wherein the coil spring has a spring tension in an absence of an externally applied axial force. The adjustable volume capacity mechanism further includes a spring force adjustment mechanism for generating an externally applied axial force to vary the spring tension of the coil spring. Increasing the spring tension of the coil spring increases the volume capacity of the inner collection chamber; whereas decreasing the spring tension of the coil spring reduces the volume capacity of the inner collection chamber. In one configuration the spring force adjustment mechanism includes: a threaded shaft inserted through a hole defined in the bottom surface of the outer collection chamber. A first end of the threaded shaft is disposed inside the outer collection chamber, while an opposite second end of the threaded shaft is disposed outside the outer collection chamber. On the first end of the threaded shaft is a supporting plate while a knob is disposed on the second end of the threaded shaft. The coil spring is disposed between the bottom surface of the inner collection chamber and the supporting plate. A knob is disposed on the second end of the threaded shaft.

While yet another aspect of the present drainage system is direction to an implantable bodily fluid drainage system including a resistance adjustment mechanism disposed at the inlet port of the collection chamber. The resistance adjustment mechanism includes a luer fitting and a selection guide received within a side slot defined in the luer fitting and having a plurality of openings defined therein of varying diameters. The selection guide is slidable within the side slot in a direction perpendicular to an axial direction of the luer fitting so that a desired one of the plural openings of the selection guide is aligned with a lumen defined through the luer fitting. Alternatively, the resistance adjustment mechanism may be configured to include a luer fitting and a collar having defined therein a plurality of openings each differing in diameter. The collar is rotatable so that a desired one of the plural openings is aligned with a lumen defined through the luer fitting.

Yet another aspect of the present implantable bodily fluid drainage system includes a gravity shutoff valve disposed at the inlet port of the collection vessel. The gravity shut off valve includes: (i) a first ball bearing secured within a complementary first hemispherical shape recess by a first compression spring; and (ii) a second ball bearing also secured within a complementary second hemispherical shape recess by a second compression spring. The second ball bearing is separated a predetermined distance from the first ball bearing in a direction of flow of the bodily fluid. The first and second compression springs compress the first and second ball bearings, respectively, in 180° opposing directions along an axis perpendicular to a direction of flow of fluid through the gravity shut off valve. The first and second hemispherical shape recesses each transitioning in the direction of flow of the bodily fluid into respective linear pathways sloping downward at approximately 45° relative to the axis perpendicular to the direction of flow of fluid through the gravity shut off valve. In the direction of flow of fluid through the gravity shut off valve, after each of the first and second hemispherical shape recesses, an axial passageway of the valve narrows to prevent the respective first and second ball bearings from passing therethrough.

### Brief Description of the Drawing

The foregoing and other features of the present invention will be more readily apparent from the following detailed description and drawings of illustrative embodiments of the invention wherein like reference numbers refer to similar elements throughout the several views and in which:
Figure 1A is a partial cross-sectional view of a rack-and-pinion overfill prevention mechanism and a coil spring operated adjustable volume capacity functionality comprising part of a bodily fluid drainage system in accordance with the present invention, wherein the drainage system is in an open state;
Figure 1B is a partial cross-sectional view of the system of Figure 1A, while in a closed state;
Figure 2A is a side view of one exemplary configuration of the luer lock of Figure 4;
Figure 2B is a top view of the luer lock of Figure 2A;
Figure 2C is a side view of another exemplary configuration of the luer lock of Figure 4;
Figure 2D is a top view of the luer lock of Figure 2C;
Figure 3 is a partial cross-sectional view of an exemplary gravity shutoff valve in Figure 4;
Figure 4 is a partial side view of the improved drainage system in accordance with the present invention.

### Detailed Description of the Invention

The present inventive volume limiting mechanisms and volume capacity adjustment mechanisms are used as part of an implantable drainage system for draining a bodily fluid, such as CSF.

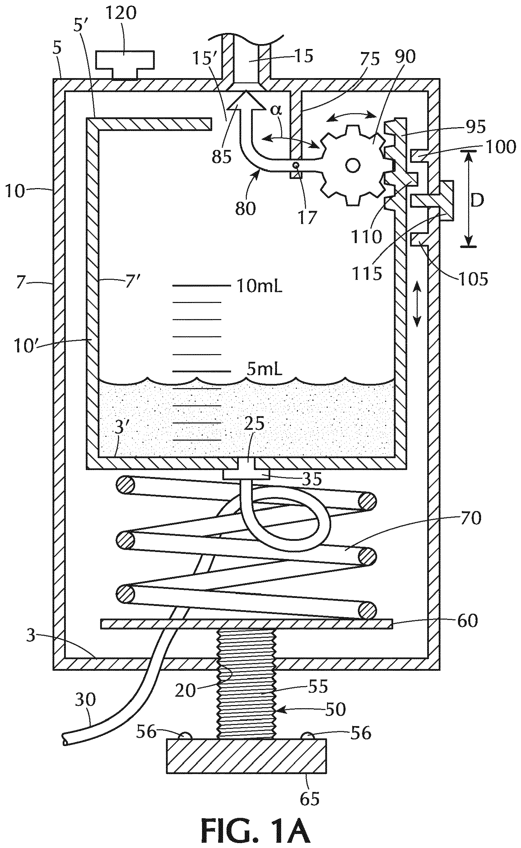

Figure 1A is a partial cross-sectional view of a first embodiment comprising a rack-and-pinion volume limiting mechanism and a coil spring adjustable volume capacity mechanism for use in an implantable bodily fluid drainage system. The implantable bodily fluid drainage system comprises a pair of collection chambers (one nested within the other) including an outer collection chamber 10 having a top surface 5, an opposite bottom surface 3 and sidewall 7 extending therebetween to form a closed outer container. Disposed within the outer collection chamber 10 is an inner collection chamber 10' having a corresponding top surface 5' an opposite bottom surface 3' and sidewall 7' extending therebetween forming a closed inner container. Inner collection chamber 10' is smaller in dimension relative to that of the outer collection chamber 10. The collection chambers 10, 10' are preferably made of a transparent material and the inner collection chamber 10' has a series of identifying volume capacity markings or lines, preferably in predetermined discrete increments (e.g., 1 ml increment hash marks) with numerical values associated with some, if not all, markings (e.g., numerical indications provided every 5 ml). Top surface 5 of outer collection chamber 10 has an associated inlet port or opening 15 defined therethrough while the top surface 5' of the inner collection chamber 10' has an associated inlet port or opening 15' defined therethrough. The two inlet ports 15, 15' in the respective outer and inner collection chambers 10, 10' are substantially aligned (but not necessarily of equal diameter) so that bodily fluid draining from an implanted catheter at a target site in the body passes through the respective inlet ports 15, 15' and is ultimately collected in the inner collection chamber 10'. No fluid is intended to accumulate in the outer collection chamber 10 outside of the inner collection chamber 10'. Thus, under proper operation all the bodily fluid from the implanted catheter is collected only in the inner collection chamber 10'.

Mounted to an interior surface of one of the walls 7 of the outer collection chamber 10 is a minimum travel limiting stop 100 and a maximum travel limiting stop 105 separated a predetermined distance (D) from one another in a longitudinal direction of the chamber 10. Each travel limiting stop 100, 105 is formed as a rib, nub or other shape projection extending radially inward into the interior of the outer collection chamber 10, preferably substantially perpendicular to sidewall 7. Along an exterior surface of the sidewall 7' of the inner collection chamber 10' is mounted a single axial movement guide 110, such as a rib, nub or other shape projection extending radially outward, preferably substantially perpendicular to sidewall 7', away from the inner collection chamber 10' towards the interior surface of the sidewall 7 of the outer collection chamber 10. Axial movement guide 110 is positioned or trapped, in an axial direction, between the minimum and maximum travel limiting stops 100, 105. Accordingly, axial displacement of the axial movement guide 110 restricts or delimits axial movement or travel of the inner collection chamber 10' relative to the outer collection chamber 10 between the minimum and maximum travel limiting stops 100, 105. The axial movement guide 110 is designed to engage with the minimum and maximum travel limiting stops 100, 105 thus restricting axial movement or travel of the inner collection chamber 10' relative to the outer collection chamber 10 to open/close the inlet port or opening 15. Arranged transversely through the wall 7 of the outer collection chamber 10 between the maximum and minimum travel limiting stops 100, 105 is a bypass switch or valve 115 that is maintained in a locked open state or position as a safety feature in case of overfill of the inner collection chamber 10'.

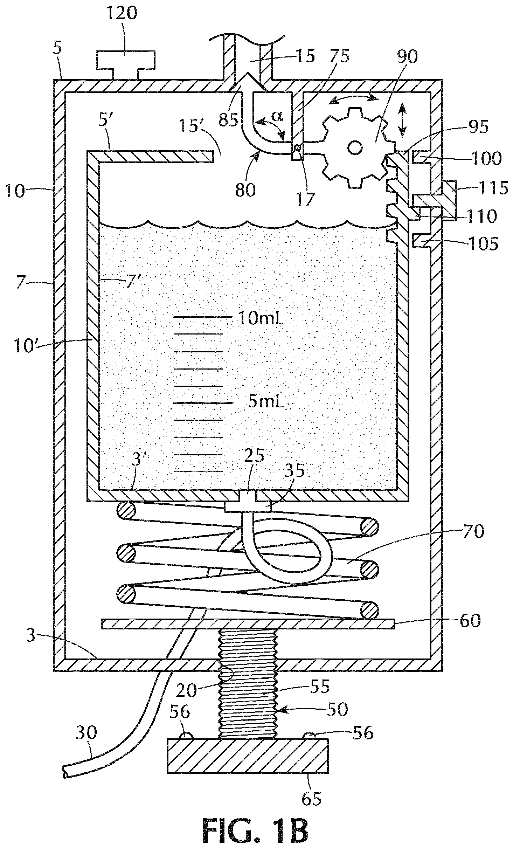

The inlet opening or port 15 of the outer collection chamber 10 is opened/closed to permit/prohibit fluid from flowing therethrough and into the inner collection chamber 10' by a rack-and-pinion mechanically operated stopper head. A fixed supporting arm 75 mounted at one end to the interior surface of the top surface 5 of the outer collection chamber 10 extends substantially perpendicularly into the interior of the outer collection chamber 10 through the inlet port or opening 15' and into the inner collection chamber 10'. A hinge 17 disposed at the opposite end of the fixed supporting arm 75 supports substantially centrally a moveable arm 80 allowing the moveable arm 80 to freely swing or rotate about the hinge 17. Moveable arm 80 has a bent or curved configuration at an angle α, preferably where 90° < α. Pinion gear 90 is disposed at one terminating end of moveable arm 80, while a stopper head 85 is configured at the opposite terminating end of the moveable arm 80. Stopper head 85 is complementary in both size and shape to that of the inlet port 15 so that when the stopper head is seated in the inlet port passage of fluid therethrough is prohibited. Pinion gear 90 matingly engages with a complementary linear rack gear 95 disposed on an interior surface of the sidewall 7' of the inner collection chamber 10'. In Figure 1A the inlet port or opening 15 of the outer collection chamber 10 is in an open state allowing the bodily fluid to pass therethrough. As fluid accumulates in the inner collection chamber 10' the inner collection chamber 10' moves axially downward relative to the outer collection chamber 10. With the axial displacement of the chambers 10, 10' relative to one another the pinion gear 90 travels along the linear rack gear 95 which, in turn, causes the moveable arm 80 to swing or rotate about the hinge 17 raising the stopper head 85 towards the inlet port or opening 15 of the outer collection chamber 10. Figure 1B shows the rack-and-pinion volume limiting mechanism of Figure 1A wherein the stopper head 85 is seated in the inlet port or opening 15 of the outer collection chamber 10 thereby closing it off to the passage of fluid therethrough.

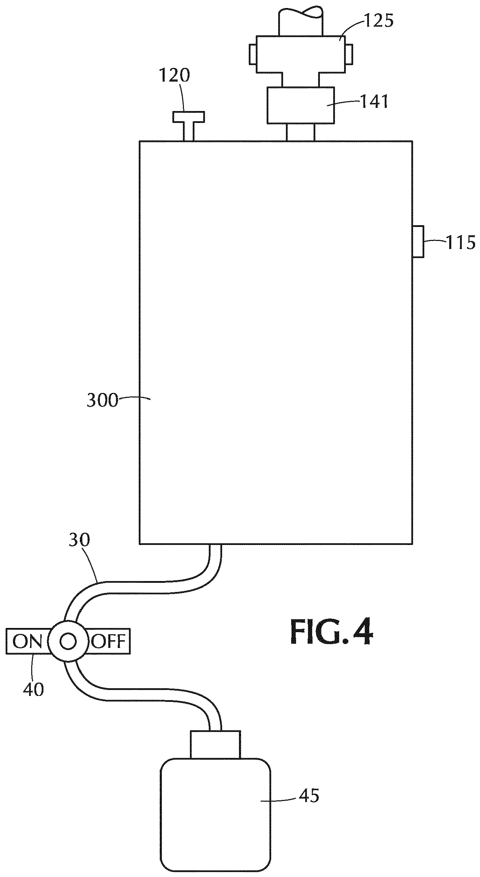

Opposite the top surface 5' of the inner collection chamber 10' is a bottom surface 3' having an outlet port or opening 25 defined therein. Tubing 30 insertable into the outlet port or opening 25 and securable in place therein via a fitter, plug or adapter 35, delivers the fluid collected in the inner collection chamber 10' to an external drainage bag 45 or other external collection vessel (Figure 4). An externally manually operated on/off stopcock or valve 40 disposed along the tubing 30 may be used to regulate the flow of fluid through the tubing 30.

Referring to Figure 1A, the volume capacity of fluid able to be collected in the inner collection chamber 10' is mechanically adjustable or controllable (increase/decreased), as desired, in accordance with the present invention via a coil spring 70 compressed axially by a mechanical spring force adjustment mechanism 50. The exemplary spring force adjustment mechanism 50 illustrated in this first embodiment of Figure 1A comprises a threaded shaft 55 having a planar supporting plate 60 at one terminating end of the threaded shaft 55 and knob or dial 65 (preferably, larger in diameter relative to the threaded shaft 55) disposed at an opposite terminating end of the threaded shaft 55. Coil spring 70 is disposed between the bottom surface 3' of the inner collection chamber 10' and the supporting plate 60.

Threaded shaft 55 passes through a slightly larger diameter hole 20 defined through the bottom surface 3 of the outer collection chamber 10 such that the supporting plate 60 is disposed within the outer collection chamber 10 while the knob or dial 65 is disposed outside of the outer collection chamber 10. Both the knob 65 and supporting plate 60 are preferably larger in diameter than the hole 20 to prohibit passage through the hole 20. One or more tactile indicators 56, e.g., bumps, projections, ribs or recesses, are preferably disposed on an under surface of the knob or dial 65 that is substantially parallel to or facing the bottom surface 3 of the outer collection chamber 10. Rotation of the knob or dial 65 increases/decreases depending on the direction of rotation (e.g., clockwise or counter-clockwise) the volume capacity in the internal collection chamber 10'. By way of illustrative example only, each revolution (360° rotation) of the knob or dial 65 clockwise/counter-clockwise, in turn, increases/decreases, respectively, the maximum volume level of fluid in the internal collection chamber 10' by a predetermined amount (e.g., approximately 50 ml) with more granular or smaller predetermined incremental adjustments (e.g., approximately 5 ml increments) provided by each tactile indicator 56.

In operation, increasing the spring tension (i.e., compression) of the coil spring 70 increases the volume capacity of bodily fluid to be accumulated in the inner collection chamber 10' before the force imposed by the accumulated fluid exceeds the counteracting opposing force exerted by the compressed coil spring 70. It is only then that the inner collection chamber 10' travels or moves axially downward toward the bottom 3 of the outer collection chamber 10. Specifically, rotation of the knob or dial 65 in a clockwise direction raises the supporting plate 60 axially upward in the outer collection chamber 10 towards the top surface 5. As the supporting plate 60 travels upward in an axial direction the strength of the external force applied to the coil spring 70 increases causing the spring tension of the coil spring 70 to increase (i.e., more compressed). In this compressed state, a greater volume of fluid may be accumulated in the inner collection chamber 10' before the force exerted by the accumulated volume of fluid exceeds, and thus counteracts, the opposing force exerted by the compressed coil spring 70 allowing the inner chamber 10' to travel axially downward relative to the outer collection chamber 10. As the volume of fluid accumulated in the inner collection chamber 10' increases further the inner collection chamber 10' lowers axially relative to the outer collection chamber 10 causing the pinion gear 90 to travel downward along the rack gear 95 while the stopper head 85 at the opposite end of the moveable arm 80 swings or rotates about hinge 17 upwards. Eventually, the inner collection chamber 10' is displaced in a longitudinal direction (lowered) sufficiently so that the stopper head 85 is seated within and closes off the inlet port or opening 15 thereby preventing overfilling.

Whereas, when the knob or dial 65 is rotated in a counter-clockwise direction the supporting plate 60 travels in a longitudinal direction downward towards the bottom surface 3 thereby reducing the external force applied to the coil spring 70 (i.e., coil spring 70 is more relaxed, less compressed, reduced spring tension). With the coil spring 70 in this more relaxed (i.e., less compressed) state, a smaller volume capacity of fluid may be accumulated in the inner collection chamber 10' before the force exerted by the accumulated fluid exceeds, and thus counteracts, the reduced opposing external force exerted by the coil spring 70 allowing the inner chamber 10' to travel axially downward relative to the outer collection chamber 10. As the volume of fluid accumulated in the inner collection chamber 10' increases still further the inner collection chamber 10' is displaced in a longitudinal direction (lowered) relative to the outer collection chamber 10 whereby the pinion gear 90 travels downward along the rack gear 95 causing the stopper head 85 at the opposite end of the moveable arm 80 to swing or rotate about hinge 17 upwards. Eventually, the inner collection chamber 10' is lowered sufficiently so that the stopper head 85 is seated within and closes off the inlet port or opening 15 preventing overfilling of the chamber.

Thus, compression of the coil spring 70 via the spring force adjustment mechanism 50 increases the amount of fluid able to be accumulated in the inner collection chamber 10' before the inner collection chamber 10' begins to move downward in a longitudinal direction thereby engaging the rack-and-pinion gear volume limiting mechanism. Alternative configurations of the spring adjustment mechanism 50 to control spring tension (i.e., the extent of compression/relaxation) of the coil spring 70 are contemplated and within the intended scope of the present invention.

Figure 4 is a partial side view of the improved drainage system in accordance with the present invention wherein, for simplicity of illustration only, the one or more collection chambers for each embodiment, as described in detail in the preceding paragraphs, has been generically represented by a single rectangular collection vessel 300. The specific configuration of the one or more collection chambers for each of the embodiments may be substituted for the generically represented rectangular collection vessel 300. For any of the foregoing described embodiments, the collection chamber preferably incorporates several backup safety features such as a hydrophobic filter 120 and/or bypass valve 115. The hydrophobic filter 120 in the top surface 5 of the outer collection chamber 5 reduces the risk of contamination of the sterile internal portions of the components of the present inventive drain system should they undesirably come into contact with the fluid being drained in the event of overfilling of the collection chamber. A bypass switch or valve 115 may be provided in the collection chamber of any of the embodiments of the drainage system described herein. The bypass switch is maintained in a locked open state or position as an additional safety feature in case of overfill of the collection chamber in which the fluid is stored.

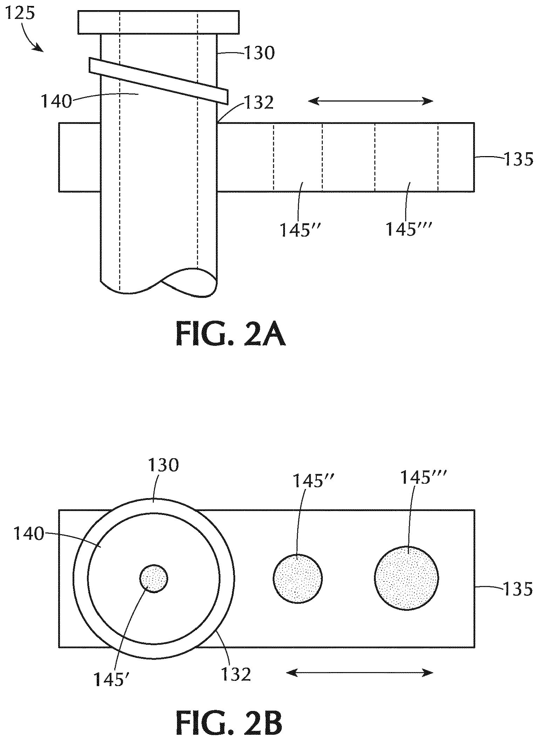

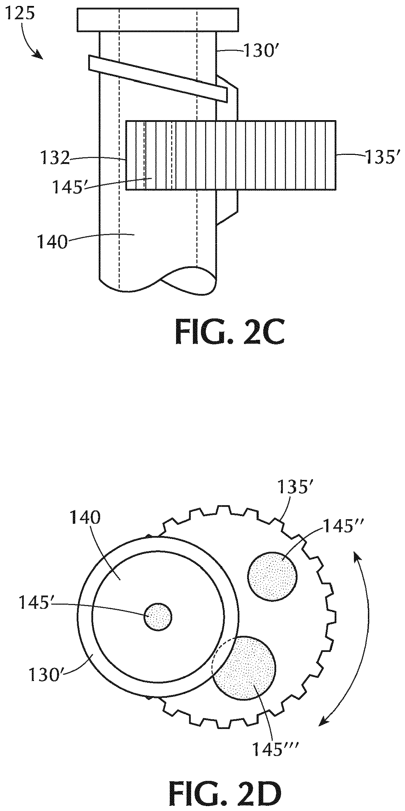

Not only may the volume capacity of the inner collection chamber 10' be adjusted or controlled in accordance with the present invention, the flow rate of the bodily fluid entering the collection chamber may also be varied, as desired. The rate of flow of fluid may be controlled or adjusted, in any of the aforementioned embodiments in accordance with the present invention, using a resistance adjustment mechanism 125 (Figure 4). Figures 2A & 2B represent side and top views of a first embodiment of the resistance adjustment mechanism 125 in Figure 4 that comprises a luer fitting 130 and a selection guide 135 received within a side slot 132 of the luer fitting. In the exemplary embodiment illustrated, selection guide 135 has three openings (145', 145", 145'") of varying diameters. One of the openings (145', 145", 145‴) is selected by sliding the selection guide 135 in a direction (as indicated by the directional arrows) perpendicular to the axial direction of the luer fitting 130 until the desired diameter opening aligns with the single uniform axial lumen 140 of the luer 130. Any number of one or more different sized diameter openings may be integrated on a single selection guide 135, otherwise a set of separate distinct guides may be utilized, with a different diameter opening associated with each separate guide in the set. An alternative embodiment of the resistance adjustment mechanism 125 is illustrated in Figures 2C & 2D including a fitting luer 130' and a rotatable collar 135'. As depicted in the top view in Figure 2D, collar 135' may be designed to have a plurality of openings (145', 145", 145‴) each differing in diameter. In the example illustrated in Figure 2D three different size diameter openings (145', 145", 145‴) are provided. Any number of one or more diameter openings having different diameters may be employed. The desired flow rate of fluid is controlled by rotating the collar 135' so that the desired size diameter opening (145', 145", 145‴) aligns with the axial lumen 140 defined in the luer fitting 130'.

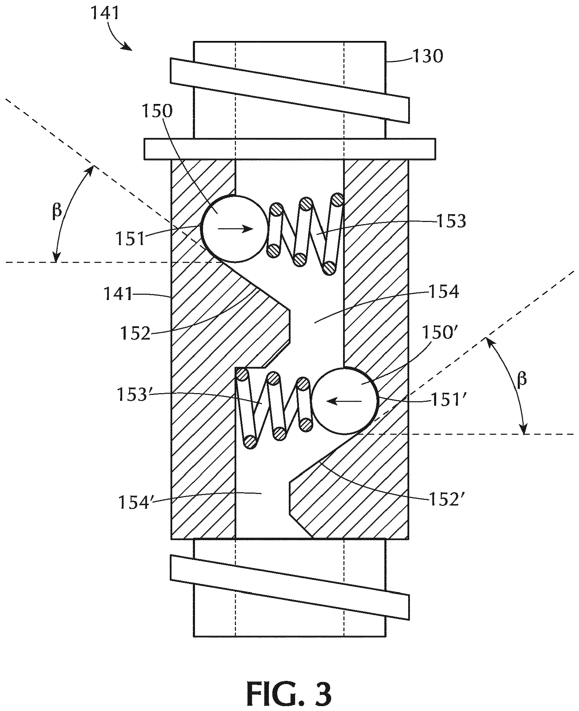

In use, the present inventive implantable drainage system may be subject to possible leakage of fluid from the inner or outer collection chambers 10', 10 with extreme movement by the patient if a supporting pole from which the collection chambers are hung should tip, tilt, lean, slope, list or be knocked over completely. It is estimated that tipping, tilting, leaning, sloping or listing of the supporting pole (with the collection chambers 10, 10' hung therefrom) greater than or equal to a predetermined angle (e.g., approximately 45°) relative to that of a vertical axis perpendicular to the floor on which the supporting pole stands would be at risk to possible leakage of fluid from the inner or outer collection chambers 10', 10. Figure 3 is an enlarged cross-sectional via of the gravity shutoff valve 141 of Figure 4. Gravity shutoff valve 141 located at the inlet port or opening 15 of the outer collection chamber 10 provides an additional safety feature to prevent or minimize leakage of the fluid from the inner and outer collection chambers 10', 10 in the event the supporting pole (and, in turn, the collection chambers 10, 10' hung therefrom) tips, tilts, leans, slopes, lists or is even knocked over greater than a desired predetermined angle (e.g., approximately 45°) relative to that of a vertical axis perpendicular to the floor on which the supporting pole stands.

Referring to the enlarged cross-sectional view in Figure 3, the gravity shutoff valve 141 includes a first ball bearing 150 and a second ball bearing 150' separated a predetermined distance from one another in an axial direction of the luer fitting 130. First ball bearing 150 is secured within a complementary hemispherical shape recess 151 by a first compression spring 153 oriented perpendicular to the axial direction of the luer fitting 130. The hemispherical shape recess 151 transitions downward in a direction of fluid flow through the luer fitting 130 into a linear pathway 152 having a downward slope defined by an angle β, preferably approximately 45°, relative to the horizontal axis (perpendicular to the axial direction of the luer fitting 130). In a similar fashion, the second ball bearing 150' is secured within a complementary hemispherical shape recess 151' by a second compression spring 153'. The hemispherical shape recess 151' transitions downward in the direction of fluid flow into a linear pathway 152' sloping downward at an angle β', preferably approximately 45°, relative to the horizontal axis (perpendicular to the axial direction of the luer fitting 130). In the luer fitting 130, downstream (in a direction of fluid flow) following each of the hemispherical shape recesses 151, 151', the axial passageway 154, 154' narrows to prevent the respective first and second ball bearings 150, 150' from passing therethrough. The first and second compression springs 153, 153' compress the first and second ball bearings 151, 151', respectively, horizontally in 180° opposing directions to address potential tipping, tilting, leaning, sloping or listing of the supporting pole (and collection chambers 10, 10' hung therefrom) in two different opposing directions. Any unwanted or undesired tipping, tilting, leaning, sloping, listing or knocking over of the supporting pole from which the collection chambers 10, 10' are hung beyond the predetermined angle (e.g., approximately 45°) results in the force imposed by one of the ball bearings 151, 151' to counterbalance the force exerted by the respective compression spring 153, 153'. As a result, one of the ball bearings 151, 151' rolls down the associated linear pathway 152, 152' and becomes seated in the associated narrow passageway 154, 154' preventing fluid leakage.

It is advantageous that the mechanical volume limiting mechanisms in accordance with the present invention do not interact with electromagnetic fields and therefore are not susceptible to interference and potential malfunction.

It is also contemplated and within the intended scope of the present invention for the resistance adjustment mechanism 125 and/or gravity shutoff valve 141 to be used with any of the embodiments discussed in any embodiment herein described and illustrated.

Thus, while there have been shown, described, and pointed out fundamental novel features of the invention as applied to a preferred embodiment thereof, it will be understood that various omissions, substitutions, and changes in the form and details of the devices illustrated, and in their operation, may be made by those skilled in the art without departing from the scope of the invention. For example, it is expressly intended that all combinations of those elements and/or steps that perform substantially the same function, in substantially the same way, to achieve the same results be within the scope of the invention. Substitutions of elements from one described embodiment to another are also fully intended and contemplated. It is also to be understood that the drawings are not necessarily drawn to scale, but that they are merely conceptual in nature. It is the intention, therefore, to be limited only as indicated by the scope of the claims appended hereto.

## Claims

1. An implantable bodily fluid drainage system, comprising:
a collection vessel; and
a mechanical volume limiting mechanism (115) for preventing overfill of the collection vessel,
**characterized in that** the collection vessel comprises:
an outer collection chamber (10) having a top surface (5), an opposite bottom surface (3) and a sidewall (7) extending therebetween; the top surface of the outer collection chamber having an inlet port (15) defined therethrough;
an inner collection chamber (10') having a top surface (5'), an opposite bottom surface (3') and a sidewall (7') extending therebetween; the inner collection chamber being disposed within the outer collection chamber; the top surface of the inner collection chamber having an inlet port (15') defined therethrough; the inlet port of the inner and outer collection chambers being aligned;
wherein the inner collection chamber is axially displaceable within the outer collection chamber;
wherein the mechanical volume limiting mechanism comprises:
a fixed supporting arm (75) mounted to an inner surface of the outer collection chamber;
a bent moveable arm (80) rotatably disposed in the inner collection chamber and supported substantially at its center by a hinge (17) to the fixed supporting arm; a stopper head (85) disposed at one terminating end of the bent moveable arm and a pinion gear (90) disposed at an opposite terminating end of the bent moveable arm; and
a rack gear (95) mounted to the inner surface of the inner collection chamber so as to engage the pinion gear and rotate the bent moveable arm about the hinge,
wherein as the bodily fluid accumulates in the inner collection chamber, the inner collection chamber travels axially downward relative to the outer collection chamber while the pinion gear on the bent moveable arm engages with the rack gear thereby raising the stopper head upwards, eventually closing off the inlet port of the outer collection chamber.

2. The implantable bodily fluid drainage system according to claim 1, further comprising an adjustable volume capacity mechanism for varying a maximum volume capacity of the inner collection chamber; the adjustable volume capacity mechanism comprising:
a coil spring (70) disposed in the outer collection chamber; the coil spring have a spring tension in an absence of an externally applied axial force;
a spring force adjustment mechanism (50) for generating an externally applied axial force to vary the spring tension of the coil spring;
wherein increased spring tension of the coil spring increases the volume capacity of the inner collection chamber; whereas decreased spring tension of the coil spring reduces the volume capacity of the inner collection chamber.

3. The implantable bodily fluid drainage system according to claim 2, wherein the spring force adjustment mechanism comprises:
a threaded shaft (55) inserted through a hole (20) defined in the bottom surface of the outer collection chamber; wherein a first end of the threaded shaft is disposed inside the outer collection chamber, while an opposite second end of the threaded shaft is disposed outside the outer collection chamber;
a supporting plate (60) disposed on the first end of the threaded shaft; wherein the coil spring is disposed between the bottom surface of the inner collection chamber and the supporting plate;
a knob (65) disposed on the second end of the threaded shaft.

4. The implantable bodily fluid drainage system according to any preceding claim, further comprising:
a maximum travel limiting stop (105) and a minimum travel liming stop (100), the maximum and minimum travel limiting stops projecting from an interior surface of the wall of the outer collection chamber;
an axial movement guide (110) projecting from an exterior surface of the wall of the inner collection chamber; axial movement of the inner collection chamber within the outer collection chamber is limited by displacement of the axial movement guide between the maximum and minimum travel limiting stops.

5. The implantable bodily fluid drainage system according to any preceding claim, further comprising a resistance adjustment mechanism (125) disposed at the inlet port of the collection chamber, wherein the resistance adjustment mechanism includes a luer fitting (130) and a selection guide (135) received within a side slot (132) defined in the luer fitting; the selection guide having a plurality of openings (145', 145", 145‴) defined therein of varying diameters; the selection guide being slidable within the side slot in a direction perpendicular to an axial direction of the luer fitting so that a desired one of the plural openings of the selection guide is aligned with a lumen (140) defined through the luer fitting.

6. The implantable bodily fluid drainage system according to any one of claims 1-4, further comprising a resistance adjustment mechanism (125) disposed at the inlet port of the collection chamber, wherein the resistance adjustment mechanism includes a luer fitting (130') and a collar (135'); wherein the collar has defined therein a plurality of openings (145', 145", 145") each differing in diameter; the collar is rotatable so that a desired one of the plural openings is aligned with a lumen (140) defined through the luer fitting.

7. The implantable bodily fluid drainage system according to any preceding claim, further comprising a gravity shutoff valve (141) disposed at the inlet port of the collection vessel, wherein the gravity shut off valve comprises:
a first ball bearing (150) secured within a complementary first hemispherical shape recess (151) by a first compression spring (153);
a second ball bearing (150') separated a predetermined distance from the first ball bearing in a direction of flow of the bodily fluid, the second ball bearing is secured within a complementary second hemispherical shape recess (151') by a second compression spring (153'); wherein the first and second compression springs compress the first and second ball bearings, respectively, in 180° opposing directions along an axis perpendicular to a direction of flow of fluid through the gravity shut off valve;
the first hemispherical shape recess transitioning in the direction of flow of the bodily fluid into a first linear pathway (152) sloping downward at approximately 45° relative to the axis perpendicular to the direction of flow of fluid through the gravity shut off valve; the second hemispherical shape recess transitioning in the direction of flow of the bodily fluid into a second linear pathway (152')sloping downward at approximately 45° relative to the axis perpendicular to the direction of flow of fluid through the gravity shut off valve;
in the direction of flow of fluid through the gravity shut off valve, after each of the first and second hemispherical shape recesses, an axial passageway (154, 154') of the valve narrows to prevent the respective first and second ball bearings from passing therethrough.

## Patentansprüche

1. Implantierbares Körperflüssigkeitsdrainagesystem, umfassend:
ein Sammelgefäß und
einen mechanischen Volumenbegrenzungsmechanismus (115) zur Verhinderung eines Überfüllens des Sammelgefäßes,
**dadurch gekennzeichnet, dass** das Sammelgefäß Folgendes umfasst:
eine äußere Sammelkammer (10) mit einer oberen Fläche (5), einer gegenüberliegenden unteren Fläche (3) und einer sich dazwischen erstreckenden Seitenwand (7), wobei die obere Fläche der äußeren Sammelkammer einen dort hindurch definierten Einlass-Port (15) hat,
eine innere Sammelkammer (10') mit einer oberen Fläche (5'), einer gegenüberliegenden unteren Fläche (3') und einer sich dazwischen erstreckenden Seitenwand (7'), wobei die innere Sammelkammer in der äußeren Sammelkammer angeordnet ist, wobei die obere Fläche der inneren Sammelkammer einen dort hindurch definierten Einlass-Port (15') hat, wobei der Einlass-Port der inneren und der äußeren Sammelkammer fluchten,
wobei die innere Sammelkammer in der äußeren Sammelkammer axial verschiebbar ist,
wobei der mechanische Volumenbegrenzungsmechanismus Folgendes umfasst:
einen feststehenden Stützarm (75), der an einer Innenfläche der äußeren Sammelkammer montiert ist,
einen gebogenen beweglichen Arm (80), der drehbar in der inneren Sammelkammer angeordnet und im Wesentlichen an seiner Mitte über ein Scharnier (17) an dem feststehenden Stützarm gestützt ist, wobei ein Stopfenkopf (85) an einem Abschlussende des gebogenen beweglichen Arms angeordnet ist und ein Ritzel (90) an einem gegenüberliegenden Abschlussende des gebogenen beweglichen Arms angeordnet ist, und
eine Zahnstange (95), die an der Innenfläche der inneren Sammelkammer montiert ist, um das Ritzel in Eingriff zu nehmen und den gebogenen drehbaren Arm um das Scharnier zu drehen,
wobei die innere Sammelkammer beim Ansammeln der Körperflüssigkeit in der inneren Sammelkammer bezüglich der äußeren Sammelkammer axial nach unten fährt, während das Ritzel an dem gebogenen beweglichen Arm mit der Zahnstange in Eingriff kommt, wodurch der Stopfenkopf nach oben angehoben wird und den Einlass-Port der äußeren Sammelkammer allmählich verschließt.

2. Implantierbares Körperflüssigkeitsdrainagesystem nach Anspruch 1, ferner umfassend einen verstellbaren Volumenkapazitätsmechanismus zum Variieren einer maximalen Volumenkapazität der inneren Sammelkammer, wobei der verstellbare Volumenkapazitätsmechanismus Folgendes umfasst:
eine in der äußeren Sammelkammer angeordnete Spiralfeder (70), wobei die Spiralfeder eine Federspannung bei einem Fehlen einer extern ausgeübten Axialkraft hat,
einen Federkraftverstellmechanismus (50) zum Erzeugen einer extern ausgeübten Axialkraft zum Variieren der Federspannung der Spiralfeder,
wobei die erhöhte Federspannung der Spiralfeder die Volumenkapazität der inneren Sammelkammer erhöht, während reduzierte Federspannung der Spiralfeder die Volumenkapazität der inneren Sammelkammer reduziert.

3. Implantierbares Körperflüssigkeitsdrainagesystem nach Anspruch 2, wobei der Federkraftverstellmechanismus Folgendes umfasst:
einen durch ein in der unteren Fläche der äußeren Sammelkammer definiertes Loch (20) eingeführten Gewindeschaft (55), wobei ein erstes Ende des Gewindeschafts in der äußeren Sammelkammer angeordnet ist, während ein gegenüberliegendes zweites Ende des Gewindeschafts außerhalb der äußeren Sammelkammer angeordnet ist,
eine an dem ersten Ende des Gewindeschafts angeordnete Stützplatte (60), wobei die Spiralfeder zwischen der unteren Fläche der inneren Sammelkammer und der Stützplatte angeordnet ist,
einen Knopf (65), der an dem zweiten Ende des Gewindeschafts angeordnet ist.

4. Implantierbares Körperflüssigkeitsdrainagesystem nach einem der vorhergehenden Ansprüche, ferner umfassend:
einen Maximalhub-Begrenzungsanschlag (105) und einen Minimalhub-Begrenzungsanschlag (100), wobei der Maximal- und der Minimalhub-Begrenzungsanschlag von einer Innenfläche der Wand der äußeren Sammelkammer vorragen,
eine Axialbewegungsführung (110), die von einer Außenfläche der Wand der inneren Sammelkammer vorragt, wobei die Axialbewegung der inneren Sammelkammer in der äußeren Sammelkammer durch Verschieben der Axialbewegungsführung zwischen dem Maximal- und dem Minimalhub-Begrenzungsanschlag begrenzt wird.

5. Implantierbares Körperflüssigkeitsdrainagesystem nach einem der vorhergehenden Ansprüche, ferner umfassend einen Widerstandsverstellmechanismus (125), der an dem Einlass-Port der Sammelkammer angeordnet ist, wobei der Widerstandsverstellmechanismus einen Luer-Anschluss (130) und eine Wahlführung (135) aufweist, die in einem in dem Luer-Anschluss definierten Seitenschlitz (132) aufgenommen ist, wobei die Wahlführung eine Vielzahl von darin definierten Öffnungen (145', 145'', 145‴) mit variierenden Durchmessern hat, wobei die Wahlführung in dem Seitenschlitz in einer senkrecht zu einer Axialrichtung des Luer-Anschlusses verlaufenden Richtung verschiebbar ist, so dass eine gewünschte der mehreren Öffnungen der Wahlführung mit einem durch den Luer-Anschluss definierten Lumen (140) fluchtet.

6. Implantierbares Körperflüssigkeitsdrainagesystem nach einem der Ansprüche 1 - 4, ferner umfassend einen Widerstandsverstellmechanismus (125), der an dem Einlass-Port der Sammelkammer angeordnet ist, wobei der Widerstandsverstellmechanismus einen Luer-Anschluss (130') und einen Bund (135') aufweist, wobei in dem Bund eine Vielzahl von Öffnungen (145', 145", 145‴) mit jeweils unterschiedlichem Durchmesser definiert sind und der Bund drehbar ist, so dass eine gewünschte der mehreren Öffnungen mit einem durch den Luer-Anschluss definierten Lumen (140) fluchtet.

7. Implantierbares Körperflüssigkeitsdrainagesystem nach einem der vorhergehenden Ansprüche, ferner umfassend ein Schwerkraft-Absperrventil (141), das an dem Einlass-Port des Sammelgefäßes angeordnet ist, wobei das Schwerkraft-Absperrventil Folgendes umfasst:
ein erstes Kugellager (150), das mittels einer ersten Druckfeder (153) in einer komplementären ersten halbkugelförmigen Aussparung (151) befestigt ist,
ein zweites Kugellager (150'), das in einer Strömungsrichtung der Körperflüssigkeit über einen vorbestimmten Abstand von dem ersten Kugellager getrennt ist, wobei das zweite Kugellager mittels einer zweiten Druckfeder (153') in einer komplementären zweiten halbkugelförmigen Aussparung (151') befestigt ist, wobei die erste und die zweite Druckfeder jeweils das erste und
das zweite Kugellager in sich 180° gegenüberliegenden Richtungen entlang einer senkrecht zu einer Flüssigkeitsströmungsrichtung durch das Schwerkraft-Absperrventil verlaufenden Achse zusammendrücken,
wobei die erste halbkugelförmige Aussparung in der Strömungsrichtung der Körperflüssigkeit in eine erste lineare Bahn (152) übergeht, die bezüglich der senkrecht zu der Flüssigkeitsströmungsrichtung durch das Schwerkraft-Absperrventil verlaufenden Achse in einem Winkel von ungefähr 45° nach unten geneigt ist, wobei die zweite halbkugelförmige Aussparung in der Strömungsrichtung der Körperflüssigkeit in eine zweite lineare Bahn (152') übergeht, die bezüglich der senkrecht zu der Flüssigkeitsströmungsrichtung durch das Schwerkraft-Absperrventil verlaufenden Achse in einem Winkel von ungefähr 45° nach unten geneigt ist,
wobei sich ein axialer Durchgang (154, 154') des Ventils in der Flüssigkeitsströmungsrichtung durch das Schwerkraft-Absperrventil nach jeder der ersten und der zweiten halbkugelförmigen Aussparung verengt, um zu verhindern, dass das erste bzw. das zweite Kugellager dort hindurchgehen.

## Revendications

1. Système de drainage de fluide corporel implantable, comprenant :
un récipient de collecte ; et
un mécanisme de limitation de volume mécanique (115) pour empêcher un débordement du récipient de collecte,
**caractérisé en ce que** le récipient de collecte comprend :
une chambre de collecte externe (10) ayant une surface supérieure (5), une surface inférieure (3) opposée et une paroi latérale (7) s'étendant entre elles ; la surface supérieure de la chambre de collecte externe ayant un orifice d'entrée (15) défini à travers elle ;
une chambre de collecte interne (10') ayant une surface supérieure (5'), une surface inférieure (3') opposée et une paroi latérale (7') s'étendant entre elles ; la chambre de collecte interne étant disposée à l'intérieur de la chambre de collecte externe ; la surface supérieure de la chambre de collecte interne ayant un orifice d'entrée (15') défini à travers elle ; les orifices d'entrée des chambres de collecte interne et externe étant alignés ;
la chambre de collecte interne pouvant être déplacée axialement à l'intérieur de la chambre de collecte externe ;
le mécanisme de limitation de volume mécanique comprenant :
un bras de support fixe (75) monté sur une surface interne de la chambre de collecte externe ;
un bras mobile plié (80) disposé à rotation dans la chambre de collecte interne et supporté sensiblement au niveau de son centre par une articulation (17) sur le bras de support fixe ; une tête de bouchon (85) disposée au niveau d'une extrémité de terminaison du bras mobile plié et un organe d'engrenage formant pignon (90) disposé au niveau d'une extrémité de terminaison opposée du bras mobile plié ; et
un organe d'engrenage formant crémaillère (95) monté sur la surface interne de la chambre de collecte interne de sorte à entrer en prise avec l'organe d'engrenage formant pignon et faire tourner le bras mobile plié autour de l'articulation,
à mesure que le fluide corporel s'accumule dans la chambre de collecte interne, la chambre de collecte interne se déplaçant axialement vers le bas par rapport à la chambre de collecte externe tandis que l'organe d'engrenage formant pignon sur le bras mobile plié entre en prise avec l'organe d'engrenage formant crémaillère, faisant monter ainsi la tête de bouchon vers le haut, fermant finalement l'orifice d'entrée de la chambre de collecte externe.

2. Système de drainage de fluide corporel implantable selon la revendication 1, comprenant en outre un mécanisme de capacité de volume ajustable pour faire varier une capacité de volume maximum de la chambre de collecte interne ; le mécanisme de capacité de volume ajustable comprenant :
un ressort hélicoïdal (70) disposé dans la chambre de collecte externe ; le ressort hélicoïdal ayant une tension de ressort en l'absence d'une force axiale appliquée de l'extérieur ;
un mécanisme d'ajustement de force de ressort (50) pour produire une force axiale appliquée de l'extérieur pour faire varier la tension de ressort du ressort hélicoïdal ;
une tension de ressort accrue du ressort hélicoïdal faisant augmenter la capacité de volume de la chambre de collecte interne ; tandis qu'une tension de ressort diminuée du ressort hélicoïdal réduit la capacité de volume de la chambre de collecte interne.

3. Système de drainage de fluide corporel implantable selon la revendication 2, le mécanisme d'ajustement de force de ressort comprenant :
un arbre fileté (55) inséré à travers un trou (20) défini dans la surface inférieure de la chambre de collecte externe ; une première extrémité de l'arbre fileté étant disposée à l'intérieur de la chambre de collecte externe,
tandis qu'une deuxième extrémité opposée de l'arbre fileté est disposée à l'extérieur de la chambre de collecte externe ;
une plaque de support (60) disposée sur la première extrémité de l'arbre fileté ; le ressort hélicoïdal étant disposé entre la surface inférieure de la chambre de collecte interne et la plaque de support ;
une molette (65) disposée sur la deuxième extrémité de l'arbre fileté.

4. Système de drainage de fluide corporel implantable selon l'une quelconque des revendications précédentes, comprenant en outre :
une butée de limitation de déplacement maximum (105) et une butée de limitation de déplacement minimum (100), les butées de limitation de déplacement maximum et minimum faisant saillie à partir d'une surface intérieure de la paroi de la chambre de collecte externe ;
un guide de mouvement axial (110) faisant saillie à partir d'une surface extérieure de la paroi de la chambre de collecte interne ; un mouvement axial de la chambre de collecte interne à l'intérieur de la chambre de collecte externe étant limité par un déplacement du guide de mouvement axial entre les butées de limitation de déplacement maximum et minimum.

5. Système de drainage de fluide corporel implantable selon l'une quelconque des revendications précédentes, comprenant en outre un mécanisme d'ajustement de résistance (125) disposé au niveau de l'orifice d'entrée de la chambre de collecte, le mécanisme d'ajustement de résistance comportant un raccord Luer (130) et un guide de sélection (135) reçu à l'intérieur d'une fente latérale (132) définie dans le raccord Luer ; le guide de sélection ayant une pluralité d'ouvertures (145', 145", 145‴) définies en son sein de diamètres variables ; le guide de sélection pouvant coulisser à l'intérieur de la fente latérale dans une direction perpendiculaire à une direction axiale du raccord Luer de sorte qu'une ouverture souhaitée de la pluralité d'ouvertures du guide de sélection est alignée avec une lumière (140) définie à travers le raccord Luer.

6. Système de drainage de fluide corporel implantable selon l'une quelconque des revendications 1 à 4, comprenant en outre un mécanisme d'ajustement de résistance (125) disposé au niveau de l'orifice d'entrée de la chambre de collecte, le mécanisme d'ajustement de résistance comportant un raccord Luer (130') et un collier (135') ; le collier ayant une pluralité d'ouvertures (145', 145", 145‴) définies en son sein, chacune de diamètre différent ; le collier pouvant tourner de sorte qu'une ouverture souhaitée de la pluralité d'ouvertures est alignée avec une lumière (140) définie à travers le raccord Luer.

7. Système de drainage de fluide corporel implantable selon l'une quelconque des revendications précédentes, comprenant en outre une soupape d'arrêt par gravité (141) disposée au niveau de l'orifice d'entrée du récipient de collecte, la soupape d'arrêt par gravité comprenant :
un premier roulement à bille (150) fixé à l'intérieur d'un premier renfoncement de forme hémisphérique (151) complémentaire par un premier ressort de compression (153) ;
un deuxième roulement à bille (150') séparé d'une distance prédéterminée du premier roulement à bille dans une direction d'écoulement du fluide corporel, le deuxième roulement à bille étant fixé à l'intérieur d'un deuxième renfoncement de forme hémisphérique (151') complémentaire par un deuxième ressort de compression (153') ; les premier et deuxième ressorts de compression comprimant les premier et deuxième roulements à bille, respectivement, dans des directions opposées à 180° le long d'un axe perpendiculaire à une direction d'écoulement de fluide à travers la soupape d'arrêt par gravité ;
le premier renfoncement de forme hémisphérique se prolongeant dans la direction d'écoulement du fluide corporel par un premier trajet linéaire (152) incliné vers le bas à environ 45° par rapport à l'axe perpendiculaire à la direction d'écoulement de fluide à travers la soupape d'arrêt par gravité ; le deuxième renfoncement de forme hémisphérique se prolongeant dans la direction d'écoulement du fluide corporel par un deuxième trajet linéaire (152') incliné vers le bas à environ 45° par rapport à l'axe perpendiculaire à la direction d'écoulement de fluide à travers la soupape d'arrêt par gravité ;
dans la direction d'écoulement de fluide à travers la soupape d'arrêt par gravité, après chacun des premier et deuxième renfoncements de forme hémisphérique, un passage axial (154, 154') de la soupape se rétrécissant pour empêcher les premier et deuxième roulements à bille respectifs pour empêcher de passer à travers.
